# EUROPÄISCHE PATENTSCHRIFT

(11) **EP 1 745 806 B1**
(45) Veröffentlichungstag und Bekanntmachung des Hinweises auf die Patenterteilung: **05.08.2009**
(21) Anmeldenummer: 06011332.1
(22) Anmeldetag: 01.06.2006
(51) Int. Cl.: A61L 27/50

(54) **Verformbares Implantatmaterial**
Deformable implant material
Matériau déformable pour implants

(30) Priorität: 22.06.2005 DE 102005029206
(43) Veröffentlichungstag der Anmeldung: 24.01.2007
(73) Patentinhaber: Heraeus Kulzer GmbH, 63450 Hanau (DE)
(72) Erfinder: Kühn, Klaus-Dieter, Dr., 5041 Marburg (DE); Vogt, Sebastia, Dr., 99084 Erfurt (DE)
(74) Vertreter: Kühn, Hans-Christian

(56) Entgegenhaltungen:
- EP-A- 1 462 131
- EP-A- 1 527 751
- EP-A- 1 629 796
- EP-A1- 0 366 018
- DE-A1- 3 134 152
- US-A- 5 522 894

## Beschreibung

Es wird ein verformbares Implantatmaterial beschrieben, das zur temporären Auffüllung von Wundhöhlen, insbesondere von Knochendefekten bestimmt ist.

Knochendefekte treten in der Chirurgie nach der Sanierung von Knochenzysten, nach dem Ausräumen von Tumoren und nach der chirurgischen Sanierung von Knocheninfektionen häufig auf. Die entstandenen Hohlräume sollen im Idealfall mit neugebildetem Knochen durchbaut werden, um den natürlichen Zustand wieder herzustellen. Knochengewebe durchbaut jedoch bei größeren, so genannten critical size-Defekten, nicht den Knochenhohlraum. Eine Möglichkeit zur Behandlung von Knochendefekten ist die Augmentation mit autologem Knochenmaterial. Die Menge an autologem Knochenmaterial ist naturgemäß bei jedem Patienten beschränkt und erfordert einen zusätzlichen, mit Risiken behafteten Zweiteingriff. Alternativ zu autologem Knochenmaterial können auch synthetisch zugängliche Knochenersatzmaterialien anstelle des autologen Knochenmaterials eingesetzt werden. Eine wichtige Gruppe sind die keramischen Knochenersatzmaterialien, die unter anderem in Form von Granulaten oder in Form von sogenannten Beads verwendet werden. Typische keramische Knochenersatzmaterialien sind in DE 196 14 421, DE 100 63 119, W09107357 und W02004112855 beschrieben. Problematisch ist jedoch bei Granulaten, dass die Granulate bestenfalls durch Verzahnung im Knochendefekt gegeneinander fixiert sind. Dadurch können Granula im Knochendefekt migrieren und sich ungleichmäßig im Knochendefekt verteilen. Die Knochenregeneration kann deshalb beeinträchtigt werden. Auch die Fixierung von keramischen Knochenersatzmaterialien durch Kollagenvliese oder Gelatinevliese ist nicht besonders aussichtsreich, weil diese sich nach wenigen Tagen auflösen.

Der Erfindung liegt die Aufgabe zu Grunde, ein Implantatmaterial bereit zu stellen, das zur Ausfüllung von Wundhöhlen, insbesondere von Knochendefekten, verwendet werden kann. Dieses Implantatmaterial soll so beschaffen sein, dass es nach Einbringung in Knochenkavitäten auf Grund seiner Struktur den Raum der Kavität ausfüllt ohne zusammen zu sinken. Das Implantatmaterial soll zur räumlichen Fixierung von keramischen Knochenersatzwerkstoffen geeignet sein.

Die Aufgabe der Erfindung wurde durch die Entwicklung eines verformbaren Implantatmaterials gelöst, welches dadurch charakterisiert ist, dass ein biodegradierbarer oder biokompatibler monofiler oder polyfiler Faden so ausgebildet ist, dass im Abstand von jeweils 2-30 mm entlang des Fadens jeweils eine kreisförmige Schlinge und oder mehrere kreisförmige Schlingen, die jeweils einen gemeinsamen Anfangspunkt entlang des Fadens haben, ausgebildet sind, wobei entlang des Fadens mindestens 3 aufeinanderfolgende Schlingen vorhanden sind und wobei die Schlingen entweder in im Wesentlichen kugel- oder oder walzenförmige Körper eingeschlossen sind, oder ringförmige Formkörper umschließen, die radial um die Fadenachse angeordnet sind. Unter einer Schlinge wird eine annähernd kreisförmige oder ellipsenförmige Ausbildung des Fadens verstanden.

Das Implantat ist bevorzugt aus Polyester, Polyamid, einer korrodierbaren Eisenlegierung, chirurgischem Stahl, Magnesium, Magnesiumlegierungen, Polysacchariden, Polysaccharidderivaten, Proteinen, Proteinderivaten oder aus Kombinationen dieser Materialien. Bei Verwendung von korrodierbaren Eisenlegierungen, Magnesium oder Magnesiumlegierungen ist das Implantatmaterial plastisch verformbar. Das erfindungsgemäße Implantatmaterial ist überraschend im Fall der Verwendung von Polyestern und Polyamiden elastisch verformbar.

Gegenstand der Erfindung sind nicht textile Flächengebilde in Form von Gewirken, Gestricken, Filzen und Vliesen, die Schlingenstrukturen und/oder Knoten enthalten.

Es ist vorteilhaft, dass die Schlingen entlang des Fadens verschoben werden können.

Zweckmäßig ist, dass die Schlingen gegebenenfalls verknotet sind.

Im Rahmen der Erfindung ist auch vorteilhaft, dass auf der Oberfläche des Implantatmaterials ein oder mehrere pharmazeutische Wirkstoffe aufgebracht sind. Diese Wirkstoffe können in Form von wachsartigen Wirkstoffen, wie Gentamicinpalmitat oder Gentamicinstearat, vorliegen, die ohne Verwendung von polymeren Filmbildnern haften. Es ist auch im Sinne der Erfindung, dass nichtfilmbildende, nicht adhäsive Wirkstoffe in niedermolekulare, gut haftende Wirkstoffe oder Hilfsstoffe eingeschlossen sind. Als niedermolekulare Hilfsstoffe kommen insbesondere gesättigte Fette und Fettsäuren in Betracht. Besonders bevorzugt ist dabei die Verwendung von Tripalmitin und Tristearin als Hilfsstoff. Als Wirkstoffe kommen Antibiotika, Antiphlogistika, Hormone und Bisphosphonate in Betracht.

Erfindungsgemäß ist, dass die Schlingen ringförmige Körper umschließen, die radial um die Fadenachse angeordnet sind. Diese Körper können in Form von Kugel oder Walzen vorliegen, die eine oder mehrere Bohrungen enthalten.

Erfindungsgemäß ist auch, dass die Schlingen in kugelförmige oder walzenförmige Körper eingeschlossen sind. Die kreisförmigen Schlingen können dabei in Form eines offenen Kreises vorliegen. Unter einem offenen Kreis wird ein Form ähnlich der des Buchstabens U verstanden. Die kugelförmigen oder walzenförmigen Körper können durch Pressen auf die Schlingen aufgebracht werden, so dass die Körper die Schlingen vollständig oder teilweise umschließen. Überraschend ist dabei, dass die Schlingen wirkungsvoll das Verrutschen der Körper entlang der Achse des Fadens verhindern.

Es ist zweckmäßig, dass die ringförmigen oder kugelförmigen oder walzenförmigen Körper aus β-Tricalciumphosphat, α-Tricalciumphosphat, Octacalciumphosphat, Rhenanit, Natrium-Kalium-Calciumphosphaten, Calciumsulfat-Dihydrat, Calciumcarbonat, Zirkoniumdioxid oder Kombinationen dieser Stoffe oder aus Kombinationen dieser Stoffe und organischen Stoffen aus der Gruppe der Polyester, Polyamide, Polymethacrylate, Polyacrylate, Proteine und der gesättigten Fette aufgebaut sind.

Weiterhin ist zweckmäßig, dass die ringförmigen oder kugelförmigen oder walzenförmigen Körper mindestens einen pharmazeutischen Wirkstoff aus der Gruppe der Antiinfektiva, der Antiphlogistika, der Cytostatika, der Bisphosphonate und der Wachstumsfaktoren enthalten.

Die ringförmigen oder kugelförmigen oder walzenförmigen Körper enthalten bevorzugt Antiinfektiva aus den Gruppen der Aminoglykosid-Antibiotika, der Lincosamid-Antibiotika, der Fluorchinolonantibiotika, der Streptogramin-Antibiotika, der Makrolid-Antibiotika, der Ketolid-Antibiotika, der Steroid-Antibiotika, der Oxazolidinon-Antibiotika und der Nitroimidazole.

Zweckmäßig ist, dass die ringförmigen oder kugelförmigen oder walzenförmigen Körper im wässrigen Milieu pharmazeutische Wirkstoffe abgeben.

Zweckmäßig ist mindestens drei oder mehrere ringförmige oder kugelförmige oder walzenförmige Körper vorhanden sind. Besonders bevorzugt ist ein Implantatmaterial, bei dem entlang der Fadenachse 30, 40 oder 60 Körper angebracht sind.

Die Verwendung des verformbaren Implantatmaterials erfolgt erfindungsgemäß dadurch, dass das verformbare Implantatmaterial als Medizinprodukt oder als Arzneimittel bereitgestellt wird.

### Zeichnungen:

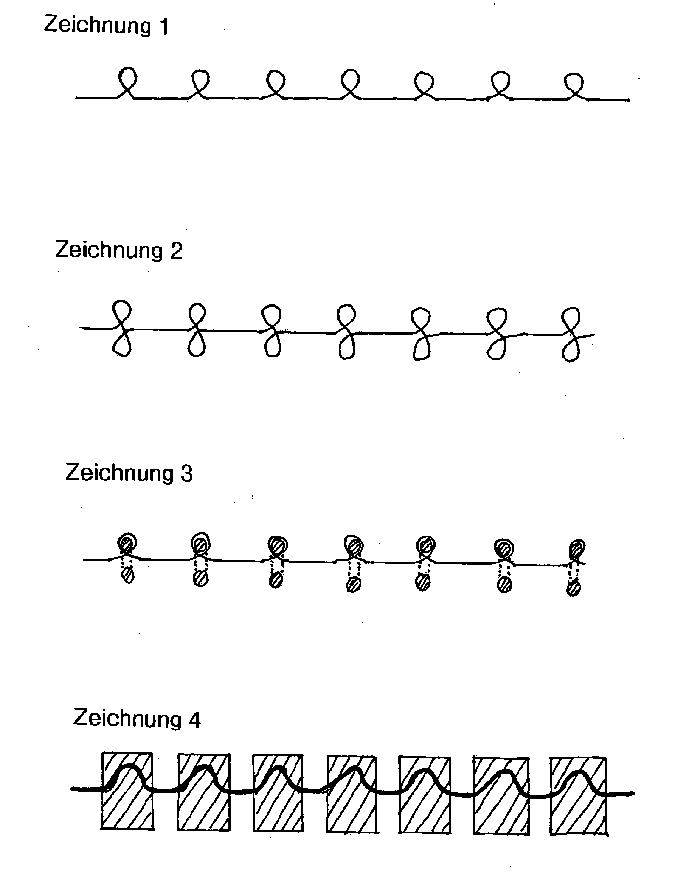

Zeichnung 1 zeigt eine erfindungsgemäße Schlingenanordnung mit 7 gleich beabstandeten Schlingen.
Zeichnung 2 zeigt eine alternative erfindungsgemäße Schlingenanordnung mit 7 gleich beabstandeten Schlingen.
Zeichnung 3 zeigt die Schlingenanordnungen der Fig. 1 und 2 mit zusätzlichen ringförmigen Körpern (schraffiert) , die von den Schlingen umschlossen werden und die radial um die Fadenachse angeordnet sind.
Zeichnung 4 zeigt eine Anordnung, bei der die Schlingen in im wesentlichen kugel- oder oder walzenförmige Körper (schraffiert) eingeschlossen sind.

Die Erfindung wird durch nachstehende Beispiele 1-5 erläutert, ohne jedoch die Erfindung einzuschränken.

### Beispiel 1

Eine Metallplatte mit 10 Stiften (Durchmesser 6mm, Abstand der Stifte 10 mm), die in einer Reihe angeordnet sind, wird als Träger zur Herstellung des Implantatmaterials eingesetzt. Ein PCL-Faden (Poly-caprolacton-co-L-lactid-Faden, USP 2-0) wird auf die Platte aufgebracht und jeweils einmal um die Stifte geschlungen. Dann wird die Platte mit dem darauf fixierten PCL-Faden im Trockenschrank auf 70 °C erhitzt und anschließend auf Raumtemperatur abgekühlt. Danach wird der PCL-Faden von den Stiften abgezogen. Der PCL-Faden hat im Abstand von jeweils 10 mm Schlingen mit einem Durchmesser von 5-6 mm. Nach dem Abkühlen sind die Schlingen bei Raumtemperatur fixiert (schematische Darstellung Zeichnung 1).

### Beispiel 2

Eine Metallplatte mit 20 Stiften (Durchmesser 6 mm, Abstand der Stifte 10 mm), wobei die Stifte in zwei gegenüberliegenden Reihen von jeweils 10 Stiften angeordnet sind und der Abstand der Reihen 12 mm beträgt, wird als Träger zur Herstellung des Implantatmaterials eingesetzt. Ein PCL-Faden (Polycaprolacton-co-L-lactid-Faden, USP 2-0) wird auf die Platte aufgebracht und jeweils einmal um die Stifte geschlungen, wobei die Fadenachse zwischen den beiden Stiftreihen liegt. Dann wird die Platte mit dem darauf fixierten PCL-Faden im Trockenschrank auf 70 °C erhitzt und anschließend auf Raumtemperatur abgekühlt und der PCL-Faden wird von den Stiften abgezogen. Der PCL-Faden hat im Abstand von jeweils 10 mm zwei sich gegenüberliegende Schlingen, die jeweils von einem gemeinsamen Punkt entlang der Fadenachse ausgehen. Die Schlingen haben einen Durchmesser von 5-6 mm. Nach dem Abkühlen sind die Schlingen bei Raumtemperatur fixiert (schematische Darstellung Zeichnung 2).

### Beispiel 3

Ein PCL-Faden (USP 2-0) mit Schlingen nach dem Beispiel 1 enthält in jeder Schlinge einen ringförmigen Körper. Die ringförmigen Körper haben eine Masse von 220 mg, einen Außendurchmesser von 6 mm und eine Höhe von 5,8 mm. Die Körper sind Komposite aus 17,31 Ma% Calciumcarbonat, 69,23 Ma% Calciumsulfat-Dihydrat, 11,80 Ma% Tripalmitin und 1,66 Ma% Gentamicinsulfat AK 600 (1,0 Ma% Gentamicinbase) (schematische Darstellung Zeichnung 3).

### Beispiel 4

Ein PCL-Faden (USP 2-0) mit Schlingen nach dem Beispiel 2 enthält in jeder Schlinge einen ringförmigen Körper. Die ringförmigen Körper haben eine Masse von 220 mg, einen Außendurchmesser von 6 mm und eine Höhe von 5,8 mm. Die Körper sind Komposite aus 17,40 Ma% Calciumcarbonat, 69,61 Ma% Calciumsulfat-Dihydrat, 11,88 Ma% Tripalmitin und 1,11 Ma% Clindamycinhydrochlorid AK 896 (1,0 Ma% Clindamycinbase).

### Beispiel 5

Auf einen PCL-Faden (USP 2-0) mit Schlingen nach dem Beispiel 1 wird mit Hilfe einer modifizierten Tablettenpresse auf jede Schlinge ausgehend von einem Pulver ein annähernd kugelförmiger Körper bei Raumtemperatur gepresst. Die Presskraft beträgt ca. 5 Tonnen. Das Pulver ist aus 17,31 Ma% Calciumcarbonat, 69,23 Ma% Calciumsulfat-Dihydrat, 11,80 Ma% Tripalmitin und 1,66 Ma% Gentamicinsulfat AK 600 (1,0 Ma% Gentamicinbase) zusammengesetzt. Die Masse der kugelförmigen Körper beträgt 250 mg (schematische Darstellung Zeichnung 4).

### Beispiel 6

Auf einen PCL-Faden (USP 2-0) wird mit Hilfe einer modifizierten Tablettenpresse auf jede Schlinge ausgehend von einem Pulver ein annähernd kugelförmiger Körper bei Raumtemperatur gepresst. Die Presskraft beträgt ca. 5 Tonnen. Der Faden wird in die Matrize so eingeführt, dass der Faden in das zu pressende Pulver nicht in der Mitte sondern in 60-70 Prozent der Füllhöhe eintritt. Das Pulver ist aus 17,31 Ma% Calciumcarbonat, 69,23 Ma% Calciumsulfat-dihydrat, 11,80 Ma% Tripalmitin und 1,66 Ma% Gentamicinsulfat (1,0 Ma% Gentamicinbase) zusammengesetzt. Beim Pressen wird eine Schlinge des PCL-Fadens in jedem einzelnen Formkörper ausgebildet. Die Masse der kugelförmigen Körper beträgt 250 mg.

## Patentansprüche

1. Verformbares Implantatmaterial zur temporären Auffüllung von Wundhöhlen
aufweisend einen biodegadierbaren oder biokompatiblen, monofilen oder polyfilen Schlingenfaden, der
im Abstand von jeweils 3-30 mm jeweils eine kreisförmige Schlinge und/oder mehrere kreisförmige Schlingen, die jeweils einen gemeinsamen Anfangspunkt entlang des Fadens haben, aufweist
wobei entlang des Fadens mindestens 3 aufeinanderfolgende Schlingen vorhanden sind,
und wobei die Schlingen entweder in im Wesentlichen kugel- oder walzenförmige Körper eingeschlossen sind, oder ringförmige Formkörper umschließen, die_radial um die Fadenachse angeordnet sind.

2. Verformbares Implantatmaterial nach Anspruch 1, aus Polyester, Polyamid, einer korrodierbaren Eisenlegierung, chirurgischem Stahl Magnesium, Magnesiumlegierungen, Polysacchariden, Polysaccharidderivaten, Proteinen, Proteinderivaten oder aus Kombinationen dieser Materialien.

3. Verformbares Implantatmaterial nach Anspruch 1 oder 2, **dadurch gekennzeichnet, dass** die Schlingen verknotet sind.

4. Verformbares Implantatmaterial nach Anspruch 1 oder 2, **dadurch gekennzeichnet, dass** auf dessen Oberfläche mindestens ein pharmazeutischer Wirkstoff aufgebracht ist.

5. Verformbares Implantatmaterial nach einem der vorangehenden Ansprüche, **dadurch gekennzeichnet, dass** die ringförmigen oder kugelförmigen oder walzenförmigen Körper aus
β-Tricalciumphosphat, α-Tricalciumphosphat, Octacalciumphosphat, Rhenanit, Natrium-Kalium-Calciumphosphaten, Calciumsulfat-Dihydrat, Calciumcarbonat, Zirkoniumdioxid oder Kombinationen dieser Stoffe oder aus Kombinationen dieser Stoffe und organischen Stoffen aus der Gruppe der Polymethacrylate, Polyacrylate, Polyester, Polyamide, Proteine, der gesättigten Fette aufgebaut sind.

6. Verformbares Implantatmaterial nach einem der vorangehenden Ansprüche, **dadurch gekennzeichnet, dass** die ringförmigen oder kugelförmigen oder walzenförmigen Körper mindestens einen pharmazeutischen Wirkstoff aus der Gruppe der Antiinfektiva, der Antiphlogistika, der Cytostatika, der Bisphosphonate und der Wachstumsfaktoren enthalten.

7. Verformbares Implantatmaterial nach einem der vorangehenden Ansprüche, **dadurch gekennzeichnet, dass** die ringförmigen oder kugelförmigen oder walzenförmigen Körper bevorzugt Antiinfektiva aus den Gruppen der Aminoglykosid-Antibiotika, der Lincosamid-Anti-biotika, der Fluorchinolonantibiotika, der Streptogramin-Antibiotika, der Makrolid-Antibiotika, der Ketolid-Antibiotika, der Steroid-Antibiotika, der Oxazolidinon-Antibiotika und der Nitroimidazole enthalten.

8. Verformbares Implantatmaterial nach einem der vorangehenden Ansprüche, **dadurch gekennzeichnet, dass** die ringförmigen oder kugelförmigen oder walzenförmigen Körper im wässrigen Milieu pharmazeutische Wirkstoffe abgeben.

9. Verformbares Implantatmaterial nach einem der vorangehenden Ansprüche, **dadurch gekennzeichnet, dass** mindestens drei oder mehrere ringförmige oder kugelförmige oder walzenförmige Körper vorhanden sind.

10. Verwendung eines verformbaren Implantatmaterials nach einem der vorstehenden Ansprüche zur Herstellung eines Mittels zur Behandlung von Knochendefekten.

## Claims

1. Mouldable implant material for filling wound cavities temporarily
exhibiting a biodegradable or biocompatible, monofilament or polyfilament loop thread which exhibits,
at a distance of 3-30 mm respectively, a circular loop and/or several circular loops which have a joint point of origin along the thread,
at least 3 consecutive loops being present along the thread,
and the loops being enclosed either in essentially spherical or roller-shaped bodies or enclosing annular moulded bodies which are arranged radially around the threat axis.

2. Mouldable implant material according to claim 1 of polyester, polyamide, a corrodible iron alloy, surgical steel, magnesium, magnesium alloys, polysaccharides, polysaccharide derivatives, proteins, protein derivatives or of combinations of these materials.

3. Mouldable implant material according to claim 1 or 2 **characterised in that** the loops are knotted.

4. Mouldable implant material according to claim 1 or 2 **characterised in that** at least one pharmaceutical active agent is applied to its surface.

5. Mouldable implant material according to one of the preceding claims **characterised in that** the annular or spherical or roller-shaped bodies consist of β-tricalcium phosphate, α-tricalcium phosphate, octacalcium phosphate, rhenanite, sodium potassium calcium phosphates, calcium sulphate dihydrate, calcium carbonate, zirconium dioxide or combinations of these substances or combinations of these substances and organic substances from the group of polymethacrylates, polyacrylates, polyesters, polyamides, proteins, of saturated fats.

6. Mouldable implant material according to one of the preceding claims **characterised in that** the annular or spherical or roller-shaped bodies contain at least one pharmaceutical active agent from the group of antiinfectives, antiphlogistics, cytostatics, bisphosphonates and growth factors.

7. Mouldable implant material according to one of the preceding claims **characterised in that** the annular or spherical or roller-shaped bodies preferably contain antiinfectives from the group of aminoglycoside antibiotics, lincosamide antibiotics, fluoroquinolone antibiotics, streptogramin antibiotics, macrolide antibiotics, ketolide antibiotics, steroid antibiotics, oxazolidinone antibiotics and nitroimidazols.

8. Mouldable implant material according to one of the preceding claims **characterised in that** the annular or spherical or roller-shaped bodies release pharmaceutical active agents in the aqueous medium.

9. Mouldable implant material according to one of the preceding claims **characterised in that** at least three or several annular or spherical or roller-shaped bodies are present.

10. Use of a mouldable implant material according to one of the preceding claims for manufacturing an agent for the treatment of bone defects.

## Revendications

1. Matériau déformable pour implants, pour comblement temporaire de plaies cavitaires, présentant un fil bouclé monofilament ou polyfilament, biodégradable ou biocompatible, qui présente à des intervalles de 3 à 30 mm une boucle circulaire et/ou plusieurs boucles circulaires qui ont chacune un point de départ commun le long du fil,
étant entendu qu'au moins trois boucles successives sont présentes le long du fil,
et étant entendu que les boucles sont incluses dans des corps sensiblement sphériques ou cylindriques ou entourent des corps moulés annulaires disposés radialement autour de l'axe du fil.

2. Matériau déformable pour implants selon la revendication 1, en polyester, polyamide, en un alliage de fer corrodable, en acier chirurgical, en magnésium, en alliages de magnésium, en polysaccharides, en dérivés de polysaccharides, en protéines, en dérivés de protéines, ou en combinaisons de ces matériaux.

3. Matériau déformable pour implants selon la revendication 1 ou 2, **caractérisé en ce que** les boucles sont nouées.

4. Matériau déformable pour implants selon la revendication 1 ou 2, **caractérisé en ce qu'**au moins un principe actif pharmaceutique est déposé à sa surface.

5. Matériau déformable pour implants selon l'une des revendications précédentes, **caractérisé en ce que** les corps annulaires ou sphériques ou cylindriques sont conçus en phosphate de β-tricalcium, en phosphate de α-tricalcium, en phosphate d'octacalcium, en rhénanite, en phosphates de sodium-potassium-calcium, en sulfate de calcium dihydraté, en carbonate de calcium, en dioxyde de zirconium ou en combinaisons de ces matériaux ou en combinaisons de ces matériaux et de matériaux organiques du groupe des polyméthacrylates, polyacrylates, polyesters, polyamides, protéines, des lipides saturés.

6. Matériau déformable pour implants selon l'une des revendications précédentes, **caractérisé en ce que** les corps annulaires ou sphériques ou cylindriques contiennent au moins un principe actif pharmaceutique du groupe des anti-infectieux, des anti-inflammatoires, des cytostatiques, des bisphosphonates et des facteurs de croissance.

7. Matériau déformable pour implants selon l'une des revendications précédentes, **caractérisé en ce que** les corps annulaires ou sphériques ou cylindriques contiennent de préférence des anti-infectieux des groupes d'antibiotiques de type aminosides, d'antibiotiques de type lincosamides, d'antibiotiques de type fluoroquinolones, d'antibiotiques de type streptogramines, d'antibiotiques de type macrolides, d'antibiotiques de type kétolides, d'antibiotiques de type stéroïdiens, d'antibiotiques de type oxazolidinones et des nitro-imidazoles.

8. Matériau déformable pour implants selon l'une des revendications précédentes, **caractérisé en ce que** les corps annulaires ou sphériques ou cylindriques libèrent des principes actifs pharmaceutiques en milieu aqueux.

9. Matériau déformable pour implants selon l'une des revendications précédentes, **caractérisé en ce que** sont présents au moins trois corps annulaires ou sphériques ou cylindriques ou plus.

10. Utilisation d'un matériau déformable pour implants selon l'une des revendications précédentes pour la fabrication d'un agent de traitement des pertes de substance osseuse.
